Europäisches Patentamt

(19) European Patent Office

Office européen des brevets

(11) Veröffentlichungsnummer: **0 041 676**
**A2**

(12) **EUROPÄISCHE PATENTANMELDUNG**

(21) Anmeldenummer: **81104173.0**

(22) Anmeldetag: **01.06.81**

(51) Int. Cl.³: **A 61 K 6/08**

(30) Priorität: **06.06.80 DE 3021380**

(43) Veröffentlichungstag der Anmeldung:
**16.12.81 Patentblatt 81/50**

(84) Benannte Vertragsstaaten:
**AT CH FR IT LI NL SE**

(71) Anmelder: **DMG - Dental-Material-Gesellschaft mbH**
**Fangdieckstrasse 61**
**D-2000 Hamburg 53(DE)**

(72) Erfinder: **Grether, Sven**
**Schillingstrasse 94**
**D-2000 Hamburg 76(DE)**

(72) Erfinder: **Behse, Ernst, Dipl.-Chem., Dr.rer.nat.**
**Oejendorfer Höhe 44**
**D-2000 Hamburg 74(DE)**

(72) Erfinder: **Mühlbauer, Ernst, Dipl.-Kaufmann**
**Dörpfeldstieg 3**
**D-2000 Hamburg 52(DE)**

(74) Vertreter: **Glawe, Richard, Dr. Dipl.-Ing.**
**Glawe, Delfs, Moll & Partner Rothenbaumchaussee 58**
**D-2000 Hamburg 13(DE)**

(54) **Material für Dentalzwecke.**

(57) Material für Dentalzwecke, das folgende Komponenten aufweist:

a) ein Kondensationsprodukt aus Diisocyanaten, Diolen oder Triolen und Methacrylsäure oder
ein Kondensationsprodukt aus Diisocyanaten und Methacrylsäurealkylestern,

b) ein Dimethacrylat oder Trimethacrylat

c) einen Methacrylsäurealkylester

sowie organische Peroxide, Polymerisationsinhibitoren Füllstoffe, Verdickungsmittel, UV-Stabilisatoren und Farbstoffe.

Beschreibung

Die Erfindung betrifft ein Material für Dentalzwecke.

Für die Herstellung von für Zahnersatz geeigneten Massen werden im allgemeinen Kunststoffe nach dem Pulver-Flüssigkeitsverfahren verwendet, vgl. DE-OS 27 49 564. Hierzu werden die Massen kurz vor ihrer Verwendung, d.h. im allgemeinen in zahntechnischen Laboratorien, aus festen Komponenten sowie flüssigen polymerisierbaren oder polykondensierbaren Komponenten zu einem Teig vermischt. Dieser Teig kann nach einer bestimmten Durchweichungsbzw. Anquellzeit gemäß üblichen Techniken verarbeitet werden. Der Teig ist als Kronen- und Brückenmaterial einfach handhabbar. Von Nachteil ist jedoch die relativ kurze Verarbeitungszeit, da durch fortwährendes Verdunsten von in dem Teig enthaltenen Komponenten, insbesondere Methacrylsäure-methylester, eine Verfestigung eintritt, die das Material rasch unverarbeitbar macht. Dies hat häufig beträchtliche Materialverluste zur Folge. Dieser Umstand verleitet den Zahntechniker zum Modellieren unter Zeitdruck. Häufig muß auch, insbesondere bei größeren Arbeiten, Pulver und Flüssigkeit erneut angerührt werden.

Andere Dentalmaterialien, die nach dem Pulver-Flüssigkeitsverfahren hergestellt werden, erfordern den Einsatz von speziellen Apparaten, z.B. Rüttlern. Da insbesondere die Mischungszeiten eingehalten werden müssen, ist eine Schulung der Zahntechniker erforderlich.

Zur Vermeidung der vorgenannten Nachteile wird erfindungsgemäß ein Material für Dentalzwecke zur Verfügung gestellt, das ein Einkomponentenmaterial darstellt und das seine

...

pastenförmige Konsistenz längere Zeit beibehält, d.h. das insbesondere 6 Monate bis mehr als 2 Jahre verarbeitbar bleibt.

Diese Aufgabe wird erfindungsgemäß dadurch gelöst, daß das Material für Dentalzwecke folgende Komponenten enthält:

a) ein Kondensationsprodukt aus einem Mol Diisocyanat, einem Mol aliphatisches Diol oder Triol und 2 Mol Methacrylsäure, wobei das Diol oder Triol 2 bis 6 Kohlenstoffatome aufweist oder Neopentylglykol, 1,4-Bishydroxymethylcyclohexan, 1,8-Octandiol oder Dihydroxydiphenylpropan ist, und/oder ein Kondensationsprodukt aus einem Mol Diisocyanat und 2 Mol Methacrylsäurealkylester, wobei die Alkoholkomponente des Methacrylsäurealkylesters von einem aliphatischen Diol oder Triol mit 2 bis 6 Kohlenstoffatomen oder Neopentylglykol, 1,4-Bishydroxydiphenylpropan 1,8-Octamdiol oder Dihydroxydiphenylpropan gebildet ist,

b) ein Dimethacrylat, dessen Alkoholkomponente von einem aliphatischen Diol oder Triol mit 2 bis 6 Kohlenstoffatomen oder Neopentylglykol, 1,4-Bishydroxymethylcyclohexan, 1,8-Octandiol oder Dihydroxydiphenylpropan gebildet ist, und/oder ein Trimethacrylat, dessen Alkoholkomponente aus 1,1,1-Trimethylolpropan besteht,

c) einen Methacrylsäurealkylester, dessen Alkylgruppe 2 bis 20 Kohlenstoffatome aufweist,

d) ein organische Peroxid,

e) Polymerisationsinhibitoren,

f) Füllstoffe, Verdickungsmittel, UV-Stabilisatoren und Farbstoffe.

...

Das Material der Erfindung ist insbesondere dadurch gekennzeichnet, daß es frei von Methacrylsäuremethylester ist.
Diese Verbindung hat sich wegen ihrer relativ größen Flüchtigkeit als ungeeignet erwiesen; darüber hinaus weist sie
den Nachteil auf, daß sie die Mundschleimhaut reizt.

Das Material der Erfindung ist weiterhin sofort, d.h. ohne
vorheriges Anmischen durch den Zahntechniker, verarbeitbar; wegen der Verwendung von höher-molekularen Monomeren
tritt insbesondere keine Verdunstung bzw. Eindicken auf.
Die sofortige Verarbeitbarkeit bedeutet weiterhin eine
beträchtliche Zeitersparnis für den Zahntechniker.

Mit dem Material der Erfindung können weiterhin sämtliche
Verarbeitungstechniken vollzogen werden, insbesondere
Modellier- und Schichttechnik sowie Küvettentechnik; dabei
sind insbesondere keine speziellen Schulungsmaßnahmen für
die Zahntechniker erforderlich.

Weiterhin gestattet das Material der Erfindung die Herstellung
eines spaltfreien Übergangs Metall/Kunststoff an dem Zahnersatz. Es weist bei richtig eingestelltem Füllstoffgehalt
einen geringen Ausdehnungskoeffizienten auf und läßt sich
sehr gut auf Hochglanz polieren.

Als Diisocyanate sind für das Material der Erfindung insbesondere die folgenden geeignet:

Tetramethylen-1,4-diisocyanat, Hexamethylen-1,6-diisocyanat,
2,2,4-Trimethylhexamethylendiisocyanat, Dodecamethylen-1,12-
diisocyanat, Diphenylmethan-4,4'-diisocyanat oder technische
Gemische desselben mit anderen Isomeren oder Triisocyanaten
sowie 2,4- oder 2,6- Toluylendiisocyanat oder Gemische derselben.
Weitere geeignete Isocyanate sind in der DE-OS 27 49 564
genannt.

...

Als aliphatische Diole oder Triole können insbesondere
Monoethylenglykol, Di-, Tri- und Tetraethylenglykol,
1,2-oder 1,3-Propandiol, 1,2-,1,3-,1,4-oder 2,3- Butandiol,
1,5-Pentandiol sowie 1,6-Hexandiol,1,8-Octandiol, Neopentylglykol, 1,4-Bishydroxymethylcyclohexan,2-Methyl-1,3-propandiol und Dihydroxydiphenylpropan eingesetzt werden.

Als Methacrylsäurealkylester können insbesondere diejenigen
Verbindungen verwendet werden, deren Alkoholkomponente 2-18,
z B.2,4,12 oder 18 Kohlenstoffatome aufweist.

Geeignete organische Peroxide sind solche, die bei Raumtemperatur mit den Einzelkomponenten des Dentalmaterials der
Erfindung nicht reagieren. Diese Reaktion soll sich erst etwa
bei 95°C, d.h. bei der üblicherweise in zahntechnischen
Laboratorien verwendeten Polymerisationstemperatur, ergeben;
bei tieferen Reaktionstemperaturen würde die Polimerisation
zu langsam ablaufen. Typische, für Dentalmaterialien bekannte
Peroxide sind Dibenzoylperoxid und Dilauroylperoxid. Für das
Dentalmaterial ist die Dilauroylperoxid besonders bevorzugt,
weil Dibenzoylperoxid zu einer kürzeren Lagerfähigkeit des
Dentalmaterials der Erfindung führt und zudem ein sprödes
Polymerisat ergeben kann. -

Weiterhin enthalten die Materialien der Erfindung Polymerisationsinhibitoren, insbesondere für diesen Zweck an sich bekannte
Phenole oder Hydroxyphenole wie p-Methoxyphenol und 2,6-Di-tert.-
butyl-p-cresol.

Schließlich können dem Material der Erfindung Füllstoffe zugesetzt werden, z.B. gemahlenes Quarz, Glas, silanisiertes
amorphes $SiO_2$, gegebenenfalls einpolymerisiert in eine
Methacrylharzmatrix, sowie UV-Stabilisatoren und übliche
Farbstoffe. Der Gehalt an Füllstoffen in dem Material der

...

Erfindung kann über weiter Bereiche schwanken und ist insbesondere von deren Dichten abhängig.

Die Mengen für die Zusätze bewegen sich im üblichen Rahmen,
so wird z.B. das Peroxid in Mengen zwischen 1 und 1,5 Gewichts-%
und der UV-Stabilisator in Mengen von bis zu 1 Gewichts-% zugesetzt, bezogen auf eingesetztes Monomer. Die Füllstoffe bzw.
Verdickungsmittel werden in einer Menge von 10 - 90 Gew.-%,
bezogen auf das Gesamtgewicht des Dentalmaterials zugesetzt.

Gemäß einer bevorzugten Ausführungsform enthält das Dentalmaterial der Erfindung 10 - 90, insbesondere 70 - 90 Gew.-Teile
der Komponente a), 1 - 35, insbesondere 5 - 20 Gew.-Teile der
Komponente b), 2 - 25, insbesondere 5 - 20 Gew.-Teile der
Komponente c), 0,1 - 3, insbesondere 0,3 - 3 Gew.-Teile der
Komponente d) und 0,01 - 0,7, insbesondere 0,05 - 0,7 Gew.-
Teile der Komponente e); die übrigen Zusätze werden nach den
oben angegebenen Prozentzahlen zugesetzt. Besonders bevorzugt
ist eine Rezeptur mit 80 Gew.-Teilen der Komponente a), 10
Gew.-Teilen der Komponente b), 10 Gew.-Teilen der Komponente
c), 1,3 - 1,5 Gew.-Teilen der Komponente d) und 0,06 - 0,20
Gew.-Teilen der Komponente e).

Als Füllstoff für das Dentalmaterial der Erfindung kann ein
mit amorphem $SiO_2$ gefülltes Splitterpolymerisat auf der Basis
der Komponenten a), b) und/oder c) oder im Dentalbereich
üblicher Monomere eingesetzt werden.

Weitere Vorteile und bevorzugte Ausführungsformen ergeben
sich aus den folgenden Beispielen.

...

(1)

| Substanz | Gewichtsteile |
|---|---|
| 7,7,9-Trimethyl-4,13-dioxo-3,14-dioxa-5, 12-diazahexadecan-1,16-diol-dimethacrylat * | 80,0 |
| Butan-1,4-diol-dimethacrylat | 10,0 |
| Methacrylsäure-ethylester | 10,0 |
| 2,6-Di-tert.butyl-4-methylphenol | 0,09 |
| UV-Absorber | 1,0 |
| Dilauroylperoxid | 1,5 |

*) Hersteller: Röhm-Chemie Darmstadt, Typ: PLEX 6661

100 g dieser bei Raumtemperatur unbegrenzt haltbaren monomeren Mischung werden in einem Laborkneter mit 12 g einer mit γ-Methacryloxypropyltrimethoxysilan behandelten handelsüblichen Aerosils (Hersteller: Degussa Typ R 972) und 14 g eines Splitterpolymerisats mit einer Korngröße von weniger als 90 µ unter Vakuum zu einer Paste gemischt. Das Splitterpolymerisat wurde durch Thermopolymerisation von 58 Gewichtsteilen handelsüblichen hydrophobem Aerosil (Typ R 972) und 42 Gewichtsteilen einer Mischung verschiedener Dimethacrylate mit Molekulargewichten zwischen 212 und 344 erhalten.

Die erhaltene gebrauchsfertige Paste ist bei Raumtemperatur über zwei Jahre lang haltbar.

...

Als Hals-, Dentin- und Schneidemasse eingefärbt, läßt sic
sich nach den in der Zahntechnik üblichen Arbeitsweisen
(Modellier- und Schichttechnik, Küvettentechnik) für Kronen-
und Brückenmaterialien verarbeiten. Die Standfestigkeit der
Pasten ist sehr gut, so daß die Schichtungen der einzelnen
Massen in einem Arbeitsgang durchgeführt werden können.
Bei der üblichen Heiß-Druck-Polymerisation (96° C heißes
Wasser, 5 bar Druck, 10 - 15 Minuten Polymerisationsdauer)
werden Verblendungen für Edelmetall-Brücken erhalten, die
nach Beschleifen und Polieren eine hervorragende Zahnähnlichkeit aufweisen. Gegenüber den herkömmlichen Kronen-
und Brückenmaterialien auf der Basis von monomerem Meth-
acrylsäure-methylester/ Perlpolymerisat zeigen die erfindungsgemäß hergestellten Pasten nach einer Heiß-Druck-Polymerisation größere Härte und sehr viel geringeren Abrieb,
aufgrund der amorphen Struktur des Füllstoffs der Pasten
sind die polymerisierten Verblendungen zudem dauerhaft
hochglanzpolierbar. Wegen des hohen Füllstoffgehaltes der
Pasten wird im Polymerisat ein geringerer thermischer Ausdehnungskoeffizient als bei herkömmlichen Kronen- und Brükken-Materialien gemessen sowie ein spaltfreier Übergang
Metall / Kunststoff beobachtet.

| Physikalische Werte | erfindungsgemäß | Stand der Technik |
|---|---|---|
| Druckfestigkeit | ca. 3600 Kp/cm² | bis zu 1500 Kp/cm² |
| Biegebruchfestigkeit | 90-100 N/mm² | 90-110 N/mm² |

Bei Normalprüfkörpern (Säulen von 12 mm Höhe, 6 mm Durchmesser) aus Polymerisaten von Methacrylsäure-methylester wird
bei einer Belastung von ca. 1100 Kp/cm² ein irreversibles
"Fließen" (Creep) gemessen. Der Prüfkörper schrumpft dabei
um mehrere Millimeter, bis er unter erhöhter Belastung
schließlich reißt und zersplittert.

...

Diesem Vorgang entspricht ein kräftiges Zubeißen eines Patienten, was mit der Zeit zur Lockerung der Verblendung von den mechanischen Retentionen einer Edelmetall-Brücke führt und mehrere negative Konsequenzen nach sich zieht. So werden Verfärbungen durch Bildung und Vergrößerung des Randspaltes zwischen Metall und Kunststoff beobachtet, was schließlich zum Ablösen und Abfallen der Verblendung führt.

Bei den erfindungsgemäß hergestellten Materialien sind die Druckfestigkeiten sehr viel höher, die unter Druck gemessene Schrumpfung ist viel geringer, die Normprüfkörper formen sich nach Entlastung weitgehend in die ursprünglichen Dimensionen zurück.

(2)

| Substanz | Gewichtsteile |
|---|---|
| 7,7,9-Trimethyl-4, 13-dioxo-3, 14-dioxa-5, 12-diazahexadecan -1, 16-diol-dimethacrylat | 80,0 |
| Triethylenglycoldimethacrylat | 8,0 |
| Methacrylsäure-n-butylester | 12,0 |
| 2,6-Di-tert.-butyl-4-methylphenol | 0,15 |
| UV-Absorber | 1,0 |
| Dilauroylperoxid | 1,3 |

Zu 100 g dieser flüssigen Harzmischung werden 14 g hochdisperse Kieselsäure (Typ R 972) und 120 g Natriumaluminiumsilikat (Hersteller: Degussa, Typ Transpafill, zu 4 % silianisiert mit $\gamma$-Methacryloxypropyltrimethoxysilan) gegeben und im Laborkneter in bekannter Weise zu einer homogenen Paste verarbeitet. Nach Zugabe von Farbpigmenten werden die in der Zahntechnik verwendeten Zahnfacetten hergestellt, indem man die Paste in vorgefräste Metallformen gibt, diese dann mechanisch preßt und bei 120° C. im Wärmeschrank polymerisiert.

...

Dabei kann es zweckmäßig sein, zur Einfärbung besonders der farbintensiven Pasten (z.B. Farbe 27, aus einem Farbstoffpulver und der oben aufgeführten Harzmischung ein Farbpastenkonzentrat zu kneten, das zu der oben genannten, noch farblosen Paste gegeben und homogen eingearbeitet wird.

Die Lagerstabilität dieser Paste überschreitet 2 Jahre.

(3)

| Substanz | Gewichtsteile |
|---|---|
| 7,7,9-Trimethyl-4, 13-dioxo-3, 14-dioxa-5, 12-diazahexadecan-1, 16-diol-dimethacrylat | 70,0 |
| Methacrylsäure-cyclohexylester | 10,0 |
| 2-Ethyl-2-hydroxymethyl-propandiol-1,3-trimethacrylat | 2,0 |
| Methacrylsäure-dodecylester | 18,0 |
| 2,6-Di-tert.-butyl-4-methylphenol | 0,1 |
| Dilauroyl-peroxid | 1,5 |

100 g dieser Harzmischung werden mit 10 g handelsüblichem, zu 10 Gew.-% silanisiertem hydrophilen Aerosil (Typ R 972) gegeben und mit 156 g eines Splitterpolymerisats, das zahnähnlich eingefärbt ist, zu einer Paste verarbeitet und mit Hilfe eine Drei-Walzen-Stuhles homogenisiert.

Das Splitterpolymerisat wurde durch Thermopolymerisation von 11 Gew.-Teilen silansierten Kieselgels (Hersteller: Grace GmbH, Worms, Typ Siloid 244), 1 Gew.-Teil handelsüblichen Aerosils (Typ R 972) und 11 Teile einer Mischung o.g. Dimethacrylate (Beispiel 1) erhalten.

In einer Heiß-Druck-Polymerisation werden aus den erfindungsgemäß hergestellten Pasten in geeigneten Vorrichtungen Kunststoffzähne hergestellt, die sich durch große Abriebfestigkeit,

...

dauerhafte Hochglanzpolitur und Elastizität auszeichnen.
Durch entsprechende Schichtungen werden porzellanähnliche
Zähne erreicht

(4)

| Substanz | Gewichtsteile |
|---|---|
| 7,7,9-Trimethyl-4, 13-dioxo-3, 14-dioxa-5, 12-diazahexadecan-1, 16-diol-dimethacrylat | 60,0 |
| Methacrylsäure-cyclohexylester | 10,0 |
| 1,1,1-Trimethylolpropantrimethacrylat | |
| Methacrylsäure-dodecylester | 18,0 |
| 2,6-Di-tert.-butyl-4-methylphenol | 0,1 |
| Dilauroyl-peroxid | 1,5 |

100 g dieser Harzmischung werden mit 10 g handelsüblichem, zu
10 Gew.-% silanisiertem hydrophilen Aerosil (Typ R 972) gegeben und mit 316 g eines feinstteiligen Bariumglases (Hersteller: Fa. Schott, Mainz, Typ GM 27884 KG) zu einer Paste
verarbeitet.

Die Paste wird mit Hilfe einer Mischmaschine zahnähnlich eingefärbt und als temporäres Kronen- und Brückenmaterial verwendet,
indem man auf einem Modell die betreffenden Krone oder Brücke
modelliert und sie anschließend in einem Druck-Polymerisationsgerät aushärtet. Der Vorteil dieses Materials liegt darin, daß
während der Tragezeit von bis zu 6 Wochen keine Abrasion festgestellt wird und sich somit für den Patienten keine Bißveränderungen ergeben.

PATENTANWÄLTE
ZUGELASSENE VERTRETER BEIM EUROPÄISCHEN PATENTAMT

RICHARD GLAWE
DR - ING

KLAUS DELFS
DIPL - ING

ULRICH MENGDEHL
DIPL.-CHEM DR RER NAT.

WALTER MOLL
DIPL.-PHYS. DR. RER. NAT.
ÖFF. BEST. DOLMETSCHER

HEINRICH NIEBUHR
DIPL.-PHYS. DR. PHIL. HABIL.

8000 MÜNCHEN 26
POSTFACH 37
LIEBHERRSTR. 20
TEL. (089) 22 65 48
TELEX 52 25 05 SPEZ

2000 HAMBURG 13
POSTFACH 25 70
ROTHENBAUM-
CHAUSSEE 58
TEL. (040) 4 10 20 08
TELEX 21 29 21 SPEZ

HAMBURG

DMG-Dental-Material-
Gesellschaft mbH

Material für Dentalzwecke.

p 9964/80    M/bo

Patentansprüche

1. Material für Dentalzwecke, gekennzeichnet durch folgende
Komponenten:

a) ein Kondensationsprodukt aus einem Mol Diisocyanat,
einem Mol aliphatisches Diol oder Triol und 2 Mol
Methacrylsäure, wobei das Diol oder Triol 2 bis 6
Kohlenstoffatome aufweist oder Neopentylglykol,
1,4-Bishydroxymethylcyclohexan, 1,8-Octandiol oder
Dihydroxydiphenylpropan ist, und/oder ein Kondensationsprodukt aus einem Mol Diisocyanat
und 2 Mol Methacrylsäurealkylester, wobei die Alkoholkomponente des Methacrylsäurealkylesters von einem
aliphatischen Diol oder Triol mit 2 bis 6 Kohlenstoffatomen oder Neopentylglykol, 1,4-Bishydroxymethylcyclohexan, 1,8-Octandiol oder Dihydroxydiphenylpropan
gebildet ist,

b) ein Dimethacrylat, dessen Alkoholkomponente von einem
aliphatischen Diol oder Triol mit 2 bis 6 Kohlenstoffatomen oder Neopentylglykol, 1,4-Bishydroxymethylcyclohexan, 1,8-Octandiol oder Dihydroxydiphenylpropan
gebildet ist; und/oder ein Trimethacrylat, dessen Alkoholkomponente aus
1,1,1-Trimethylolpropan besteht,

... 2

c) einen Methacrylsäurealkylester, dessen Alkylgruppe
2 bis 20 Kohlenstoffatome aufweist,

d) ein organisches Peroxid,

e) Polymerisationsinhibitoren,

f) Füllstoffe, Verdickungsmittel, UV-Stabilisatoren
und Farbstoffe.

2. Material nach Anspruch 1, dadurch gekennzeichnet, daß
die Komponenten a) bis e) in folgender Zusammensetzung
vorhanden sind:

10 bis 90 Gewichtsteile der Komponente a)
1 bis 35 Gewichtsteile der Komponente b)
2 bis 25 Gewichtsteile der Komponente c)
0,1 bis 3 Gewichtsteile der Komponente d)
0,01 bis 0,7 Gewichtsteile der Komponente e)

3. Material nach Anspruch 1, dadurch gekennzeichnet, daß
die Komponenten a) bis e) in folgender Zusammensetzung
vorhanden sind:

70 bis 90 Gewichtsteile der Komponente a)
5 bis 20 Gewichtsteile der Komponente b)
5 bis 20 Gewichtsteile der Komponente c)
0,3 bis 3 Gewichtsteile der Komponente d)
0,05 bis 0,7 Gewichtsteile der Komponente e)

4. Material nach Anspruch 1, dadurch gekennzeichnet, daß
man als organisches Peroxid Dilauroylperoxid einsetzt.

-3-

5. Material nach Anspruch 1, dadurch gekennzeichnet, daß der Füllstoff ein mit amorphem $SiO_2$ gefülltes Splitterpolymerisat auf der Basis der Komponenten a), b) und/oder c) oder im Dentalbereich üblicher Monomeren ist.

-3-